(19) Europäisches Patentamt
European Patent Office
Office européen des brevets

(11) Veröffentlichungsnummer: **0 142 785**
**B1**

(12) # EUROPÄISCHE PATENTSCHRIFT

(45) Veröffentlichungstag der Patentschrift: **18.03.87**

(51) Int. Cl.⁴: **C 07 C 161/02**, A 61 K 31/26

(21) Anmeldenummer: **84113507.2**

(22) Anmeldetag: **08.11.84**

(54) **Trihaloallylthiocyanate, ein Verfahren zu ihrer Herstellung und ihre Verwendung in mikrobiziden Mitteln.**

(30) Priorität: **17.11.83 DE 3341556**

(43) Veröffentlichungstag der Anmeldung: **29.05.85 Patentblatt 85/22**

(45) Bekanntmachung des Hinweises auf die Patenterteilung: **18.03.87 Patentblatt 87/12**

(84) Benannte Vertragsstaaten: **AT BE DE FR GB IT NL SE**

(56) Entgegenhaltungen: **US-A-3 170 942**

**Die Akte enthält technische Angaben, die nach dem Eingang der Anmeldung eingereicht wurden und die nicht in dieser Patentschrift enthalten sind.**

(73) Patentinhaber: **BAYER AG, Konzernverwaltung RP Patentabteilung, D-5090 Leverkusen 1 Bayerwerk (DE)**

(72) Erfinder: **Schmitt, Hans- Georg, Dr., Gustav-Freytag- Strasse 2, D-5090 Leverkusen (DE)**
Erfinder: **Paulus, Wilfried, Dr., Deswatinesstrasse 90, D-4150 Krefeld (DE)**
Erfinder: **Genth, Hermann, Dr., Am Heckerhof 60, D-4150 Krefeld (DE)**

**EP 0 142 785 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1987

**Beschreibung**

Die Erfindung betrifft neue Trihaloallylthiocyanate, ein Verfahren zu ihrer Herstellung sowie ihre Verwendung in mikrobiziden Mitteln.

Es wurden neue Trihaloallylthiocyanate der Formel

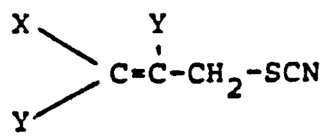

in der
X für Brom, Chlor oder lod und
Y für Brom oder lod stehen,
gefunden.

Die neuen Trihaloallylthiocyanate haben als Wirkstoffe in mikrobiziden Mitteln eine überragende und nicht zu erwartende Wirkung.

Beispielsweise seien die folgenden Trihaloallylthiocyanate genannt:

2,3,3-Triiodallylthiocyanat, 2,3-Dibrom-3-chlor-allyl-thiocyanat, 2,3-Diiod-3-chlor-allylthiocyanat, 2,3,3-Tribrom-allylthlocyanat, 2,3-Diiod-3-brom-allylthio-cyanat, 2,3-Dibrom-3-iod-allylthiocyanat.

Bevorzugte Trihaloallylthiocyanate sind erfindungsgemäß beispielsweise 2,3,3-Triiodallylthiocyanat, 2,3-DDibrom-3-iod-allylthiocyanat, 2,3,3-Tribrom-allyl-thiocyanat, 2,3-Diiod-3-chlor-allylthiocyanat, 2,3-Diiod-3-brom-allylthiocyanat.

Es wurde auch ein Verfahren zur Herstellung der neuen Trihaloallylthiocyanate gefunden, das dadurch gekennzeichnet ist, daß man Trihaloallylverbindungen der Formel

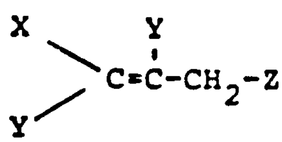

in der
X für Brom, Chlor oder lod und
Y für Brom oder lod stehen und
Z für eine nucleofuge Gruppe steht,
mit einem Thiocyanat der Formel
$M^{\oplus} SCN^{\ominus}$
in der
M Alkali oder Ammonium bedeutet,
umsetzt.

Das erfindungsgemäße Verfahren kann beispielsweise anhand der folgenden Gleichung erläutert werden:

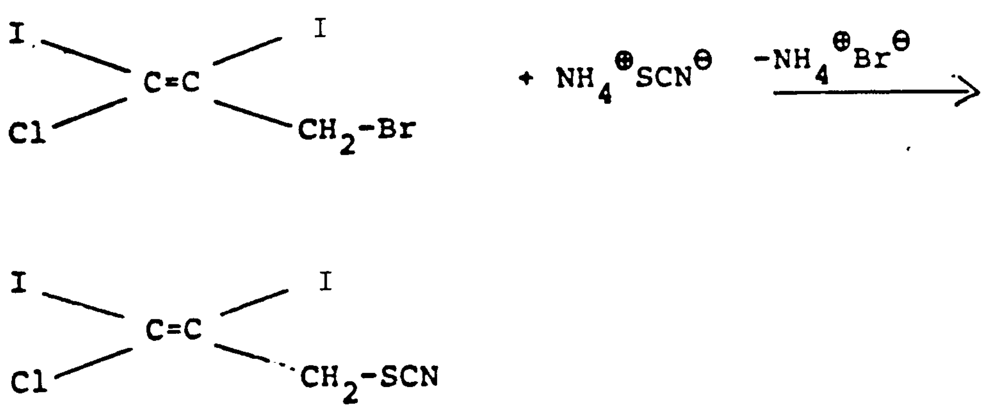

Unter Alkali im Rahmen des erfindungsgemäßen Verfahrens werden die Metalle der ersten Gruppe des Periodensystems nach Mendelejew verstanden. Beispielsweise seien Lithium, Natrium, Kalium, Rubidium und Caesium, bevorzugt Natrium und Kalium genannt.

Unter einer nucleofugen Gruppe versteht man erfindungsgemäß ein Atom oder eine Atomgruppe, die auf Grund ihrer Fähigkeit, negative Ladungen zu stabilisieren, Substitutionsreaktionen am gesättigten Kohlenstoffatom ermöglicht.

Nucleofuge Gruppen sind beispielsweise Halogene wie Fluor, Chlor, Brom und Iod, bevorzugt Chlor und Brom,

Alkylsulfonyloxygruppen wie Methylsulfonyloxy, Ethylensulfonyloxy und Arylsulfonyloxy wie Phenylsulfonyloxy, 4-Tolylsulfonyloxy.

Die als Ausgangsverbindungen eingesetzten Trihaloallylderivate sind zum Teil bekannt JP Pat. 82, 112,303, C.A. 97, 177 037 (1982) und können nach an sich bekannten Verfahren hergestellt werden. So können z.B. Trihaloallylhalogenide durch Halogenierung von Halopropargylhalogeniden erhalten werden. Sulfonsäuretrihaloallylester erhält man durch Umsetzung von Trihaloallylalkoholen mit Sulfonsäurechloriden.

Beispielsweise seien die folgenden Trihaloallylderivate genannt:

3-Chlor-2,3-diiodallylchlorid, 3-Brom-2,3-diiodallylbromid, 2,3,3-Triiodallylchlorid, 2,3,3-Tribromallylbromid und 2,3-Dibrom-3-iodallylchlorid.

Thiocyanate für das erfindungsgemäße Verfahren sind bevorzugt Natrium-, Kalium- und Ammoniumthiocyanat.

Das erfindungsgemäße Verfahren kann gegebenenfalls in Gegenwart eines Lösungs- bzw. Verdünnungsmittels durchgeführt werden. Als Lösungs- bzw. Verdünnungsmittel werden im allgemeinen polare Lösungs- bzw. Verdünnungsmittel verwendet, die sich unter den Reaktionsbedingungen nicht verändern. Beispielsweise seien die folgenden Lösungs- bzw. Verdünnungsmittel genannt: Wasser, Alkohole, wie Methanol und Ethanol, Ether, wie Dioxan oder Tetrahydrofuran, Ketone wie Aceton, Amide, wie Dimethylformamid, und Nitrile wie Acetonitril.

Das erfindungsgemäße Verfahren wird im allgemeinen im Temperaturbereich von -20°C und 100°C, bevorzugt im Bereich von 0°C bis 60°C, durchgeführt.

Im allgemeinen wird das erfindungsgemäße Verfahren bei Normaldruck durchgeführt. Es kann aber auch bei einem Über- oder Unterdruck durchgeführt werden.

Die Komponenten des erfindungsgemäßen Verfahrens werden üblicherweise in äquimolaren Mengen eingesetzt.

Zur Beschleunigung der Umsetzung kann es vorteilhaft sein einen Katalysator wie Natriumiodid der Umsetzung zuzufügen. Im allgemeinen gibt man 0,0001 bis 0,2, bevorzugt 0,001 bis 0,05 Gew.-Teile des Katalysators bezogen auf das Trihaloallylderivat zu der Umsetzung.

Das erfindungsgemäße Verfahren kann beispielsweise wie folgt durchgeführt werden. Das Thiocyanat wird gegebenenfalls in einem Lösungsmittel gelöst oder suspendiert und das Trihaloallylderivat zugegeben. Gegebenenfalls in Gegenwart eines Katalysators rührt man das Reaktionsgemisch bei der gewünschten Reaktionstemperatur.

Nach Beendigung der Umsetzung erhält man sie erfindungsgemäßen Trihaloallylthiocyanate in an sich bekannter Weise durch Einengung, Abtrennen von gebildeten Salz, Umkristallisieren oder Umfällen.

Die erfindungsgemäßen Trihaloallylthiocyanate können als Wirkstoffe zur Bekämpfung von Mikroorganismen, vorzugsweise in technischen Materialien, verwendet werden.

Technische Materialien sind erfindungsgemäß nicht lebende Materialien, die für die Verwendung in der Technik zubereitet worden sind. Beispielsweise können technische Materialien, die durch den erfindungsgemäßen Wirkstoff vor einer mikrobiellen Veränderung oder Zerstörung geschützt werden sollen, Klebstoffe, Leime, Papier und Karton, Textilen, Leder, Holz, Anstrichmittel und Kunststoffartikel, Kühlschmiermittel und andere Materialien sein, die durch Mikroorganismen zersetzt werden können. Im Rahmen der zu schützenden Materialien seien auch Teile von Produktionsanlagen, beispielsweise Kühlwasserkreisläufe genannt, die durch Mikroorganismen beeinträchtigt werden können. Im Rahmen der vorliegenden Erfindung seien als technische Materialien vorzugsweise Klebstoffe, Leime, Papier und Karton, Leder, Holz, Anstrichmittel, Kühlschmiermittel und die Kühlkreisläufe genannt.

Als Mikroorganismen, die einen Abbau oder eine Veränderung der technischen Materialien bewirken können, seien beispielsweise Bakterien, Hefen, Algen und Schleimorganismen genannt. Vorzugsweise wirken die erfindungsgemäßen Wirkstoffe gegen Bakterien, Schimmelpilze, insbesondere holzverfärbende und holzzerstörende Pilze (Basidicmyceten) sowie gegen Schleimorganismen und Algen. Es seien beispielsweise Mikroorganismen der folgenden Gattungen genannt:

Alternaria, wie Alternaria tenuis,
Aspergillus, wie Aspergillus niger,
Chaetomium, wie Chaetomium globosum,
Coniophora, wie Coniophora puteana,
Lentinus, wie Lentinus tigrinus,
Penicillium, wie Penicillium glaucum,
Polyporus, wie Polyporus versicolor,
Aureobasidium, wie Aureobasidium pullulans,
Sclerophoma, wie Sclerophoma pityophila,

Staphylococcus, wie Staphylococcus aureus,
Pseudomonas, wie Pseudomonas aeruginosa,
Escherichia, wie Escherichia coli,
ferner Grün-, Blau- und Kieselalgen.

Je nach ihrem Anwendungsgebiet können die erfindungsgemäßen Wirkstoffe in die üblichen Formulierungen überführt werden, wie Lösungen, Emulsionen, Suspensionen, Pulver, Pasten und Granulate.

Diese können in an sich bekannter Weise hergestellt werden, z.B. durch Vermischen der Wirkstoffe mit einem Streckmittel, das aus flüssigem Lösungsmittel und/oder festen Trägerstoffen besteht, gegebenenfalls unter Verwendung von oberflächenaktiven Mitteln, wie Emulgatoren und/oder Dispergiermitteln, wobei beispielsweise im Falle der Benutzung von Wasserstreckmitteln, gegebenenfalls organische Lösungsmittel wie Alkohole, als Hilfsmittel verwendet werden können.

Organische Lösungsmittel für die Wirkstoffe können beispielsweise Alkohole, wie niedere aliphatische Alkohole, vorzugsweise Ethanol oder Isopropanol, oder Benzylalkohol, Ketone wie Aceton oder Methylethylketon, flüssige Kohlenwasserstoffe, wie Benzinfraktionen, halogenierte Kohlenwasserstoffe, wie 1,2-Dichlorethan sein.

Die erfindungsgemäßen mikrobiziden Mittel enthalten den Wirkstoff im allgemeinen in einer Menge von 0,5 bis 95 Gew.-Teilen, bevorzugt von 1 bis 50 Gew.-%.

Die Anwendungskonzentration der erfindungsgemäßen Wirkstoffe richtet sich nach der Art und dem Vorkommen der zu bekämpfenden Mikroorganismen, sowie nach der Zusammensetzung des zu schützenden Materials. Die optimale Einsatzmenge kann durch Testreihen ermittelt werden.

Im allgemeinen liegen die Anwendungskonzentrationen im Bereich von 0,001 bis 5 Gew.-%, vorzugsweise von 0,01 bis 0,5 Gew.-g, bezogen auf das zu schützende Material.

Die erfindungsgemäßen neuen Wirkstoffe können auch in Mischung mit anderen bekannten Wirkstoffen vorliegen. Beispielsweise seien die folgenden Wirkstoffe genannt:. Benzylalkoholmono(Poly)hemiformal, Benzimidazolylmethylcarbamate, Tetramethyl-thiuramdisulfid, Zinksalze von Dialkyldithiocarbamaten, 2,4,5,6-Tetrachlorisophtalonitril, Thiazolylbenzimidazol, Mercaptobenzthiazol und Phenolderivate, wie 2-Phenylphenol, (2,2'-Dihydroxy-5,5'-dichlor)-diphenylmethan und 3-Methyl-4-chlor-phenol.

## Herstellungsbeispiele

### Beispiel 1

2,3,3-Triiodallylthiocyanat

9,14 g (0,113 Mol) Natriumthiocyanat werden in 200 ml Aceton gelöst; dann gibt man 51,4 g (0,113 Mol) 3-Chlor-1,1,2-triiod-1-propen hinzu. Als Katalysator fügt man außerdem noch 0,2 g Natriumiodid hinzu. Das Reaktionsgemisch wird 12 Stunden bei Raumtemperatur gerührt.

Nach Beendigung der Umsetzung wird das Reaktionsgemisch am Rotationsverdampfer eingeengt, der Rückstand in 500 ml Wasser eingerührt, die feste Ausfällung abgesaugt und Exsiccator getrocknet.

51 g (94,6 % d.Th.) hellbeige Kristalle vom Schmelzpunkt 62 bis 63° C.

IR: $\gamma_{SCN}$ 2145, 2160 $cm^{-1}$

NMR: $\gamma$ ($CDCl_3$) 4,25 ppm.

Analog werden erhalten:

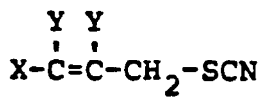

| Beispiel Nr. | X | Y | Schmp. (°C) | Ausbeute (%) | $^1$H-NMR $\delta(CDCl_3)CH_2$ (ppm) | IR $\gamma SCN$ ($cm^{-1}$) |
|---|---|---|---|---|---|---|
| 2 | Cl | Br | 38 | 82,3 | 4,22 | 2160 |
| 3 | Cl | I | 54-55 | 86,5 | 4,23 | 2152 |
| 4 | Br | Br | 43-44 | 98,5 | 4,21 | 2158 |
| 5 | Br | I | 44-45 | 99,0 | 4,20 | 2158 |
| 6 | I | Br | 60-62 | 65,3 | 4,30 | 2158 |

## Anwendungsbeispiele

### Beispiel 7

Zum Nachweis der Wirksamkeit gegen Pilze werden die minimalen Hemm-Konzentrationen (MHK) von erfindungsgemäßen Wirkstoffen bestimmt:

Ein Agar, der aus Bierwürze und Pepton hergestellt wird, wird mit erfindungsgemäßen Wirkstoffen in Konzentrationen von 0,1 mg/l bis 5000 mg/l versetzt. Nach Erstarren des Agars erfolgt Kontamination mit Reinkulturen der in der Tabelle aufgeführten Testorganismen. Nach 2-wöchiger Lagerung bei 28° C und 60 bis 70 % rel. Luftfeuchtigkeit wird die MHK bestimmt. MHK ist die niedrigste Konzentration an Wirkstoff, bei der keinerlei Bewuchs durch die verwendete Mikrobenart erfolgt, sie ist in der nachstehenden Tabelle 1 angegeben.

**Tabelle 1** MHK's in mg/l bei der Einwirkung erfindungsgemäßer

Substanzen auf Pilze

| Testorganismen | Substanz | | | | | |
|---|---|---|---|---|---|---|
| gem.Bsp. | 1 | 2 | 3 | 4 | 5 | 6 |
| Alternaria tenuis | 2 | | | | | 5 |
| Aspergillus niger | 3,5 | 50 | < 20 | 50 | < 20 | 20 |
| Aureobasidium pullulans | 1 | | | | | 5 |
| Chaetomium globosum | 5 | 50 | < 20 | 50 | < 20 | 35 |
| Coniophora puteana | 0,5 | | | | | 1 |
| Lentinus tigrinus | 0,5 | | | | | 10 |
| Penicillium glaucum | 2 | 50 | < 20 | 50 | < 20 | 10 |
| Polyporus versicolor | 0,5 | | | | | 5 |
| Sclerophoma pityophila | 0,5 | | | | | 2 |
| Trichoderma viride | 5 | | | | | 20 |

**Beispiel 8**

(Wirkung gegen Bakterien)

Ein Agar, der als Nährmedium Bouillon enthält, wird mit den in Tabelle 2 angegebenen Wirkstoffen in Konzentrationen Von 1 bis 5000 mg/l versetzt. Darauf infiziert man das Nährmedium jeweils mit Escherichia coli oder Staphylococcus aureus und hält das infizierte Medium 2 Wochen bei 28° C und 60 bis 70 % relativer Luftfeuchtigkeit. Die MHK ist die niedrigste Konzentration an Wirkstoff, bei der keinerlei Bewuchs durch die verwendete Mikrobenart erfolgt.

Die MHK-Werte sind in Tabelle 2 wiedergegeben.

**Tabelle 2**

Angabe der MHK-Werte in mg/l bei der Einwirkung der unten angegebenen Wirkstoffe auf Bakterien.

| Testorganismen | Wirkstoffe | | | | | |
|---|---|---|---|---|---|---|
| gem.Bsp. | 1 | 2 | 3 | 4 | 5 | 6 |
| Escherichia coli | 35 | 100 | 100 | 100 | 50 | 100 |
| Staphylococcus aureus | < 20 | 50 | < 20 | < 50 | < 20 | < 20 |

**Beispiel 9**

(Wirkung gegen Schleimorganismen)

Erfindungsgemäße Substanzen werden in Konzentrationen von jeweils 0,1 bis 100 mg/l in Allens Nährlösung (Arch. Mikrobiol. 17, 34 bis 53 (1952)), die in 4 l sterilem Wasser, 0,2 g Ammoniumchlorid, 4,0 g Natriumnitrat, 1,0 g Dikaliumhydrogenphosphat, 0,2 g Calciumchlorid, 2,05 g Magnesiumsulfat, 0,02 g Eisenchlorid und 1 % Caprolactam enthält, in wenig Aceton gelöst, zur Anwendung gebracht. Kurz vorher wird die Nährlösung mit Schleimorganismen (ca. $10^6$ Keime/ml), die aus bei der Polyamid-Herstellung verwendeten Spinnwasser-Kreisläufen isoliert wurden, infiziert. Nährlösungen, die die minimale Hemmkonzentration (MHK) oder größere Wirkstoffkonzentrationen aufweisen, sind nach 3-wöchiger Kultur bei Raumtemperatur noch völlig klar, d.h. die in wirkstofffreien Nährlösungen nach 3 bis 4 Tagen bemerkbare Vermehrung der Mikroben und Schleimbildung unterbleibt.

**Tabelle 3**

MHK-Werte in mg/l bei der Einwirkung der unten angegebenen Substanzen auf Schleimorganismen

| Wirkstoff gemäß Beispiel | MHK in mg/l |
|---|---|
| 1 | 0,75 |
| 6 | 3,5 |

**Beispiel 10**

Eine Mischkultur von Grün-, Blau-, Braun- und Kieselalgen (Stichococcus bacillaris Naegeli, Euglena gracilis Klebs, Chlorella pyrenoidosa Chick, Phormidium foveolarum Gomont, Oscillatoria geminata Meneghini und Phaeodactylum tricornutum Bohlin) wird unter Durchperlen von Luft in Allens Nährlösung (Arch. Mikrobiol. 17, 34 bis 53 (1952)), die auf 4 1 steriles Wasser 0,2 g Ammoniumchlorid, 4,0 g Natriumnitrat, 1,0 g Dikaliumhydrogenphosphat, 0,2 g Calciumchlorid, 2,05 g Magnesiumsulfat, 0,02 g Eisenchlorid und 1 % Caprolactam enthält, gegeben. Nach 2 Wochen ist die Nährlösung durch intensives Algenwachstum tief grünblau gefärbt. Das Absterben der Algen nach Zugabe erfindungsgemäßer Wirkstoffe erkennt man an dem Entfärben der Nährlösung.

**Tabelle 4**

Algen-abtötende Konzentrationen (mg/l) der unten angegebenen Substanzen

| Wirkstoff gemäß Beispiel | abtötende Konzentrationen in mg/l |
|---|---|
| 1 | 35 |
| 6 | 50 |

**Patentansprüche**

1. Trihaloallylthiocyanate der Formel

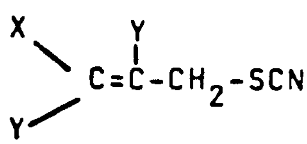

,

in der
X für Brom, Chlor oder Iod und
Y für Brom oder Iod stehen.

2. Verfahren zur Herstellung von Trihaloallylthiocyanaten, dadurch gekennzeichnet, daß man Trihaloallylverbindungen der Formel

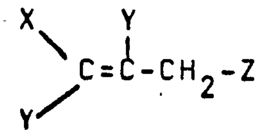

,

in der
X für Brom, Chlor oder Iod und
Y für Brom oder Iod stehen und
Z für eine nucleofuge Gruppe steht,
mit einem Thiocyanat der Formel
$M^{\oplus} SCN^{\ominus}$
in der
M Alkali oder Ammonium bedeutet,
umsetzt.

3. Verfahren nach Anspruch 2, dadurch gekennzeichnet, daß es im Temperaturbereich von -20°C bis 100°C durchgeführt wird.

4. Verfahren nach Ansprüchen 2 und 3, dadurch gekennzeichnet, daß als Katalysator Natriumiodid eingesetzt wird.

5. Mikrobizide Mittel enthaltend Trihaloallylthiocyanat der Formel

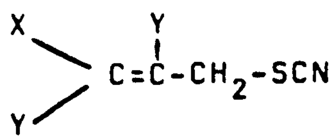

,

in der
X für Brom, Chlor oder Iod und
Y für Brom oder Iod stehen.

6. Verwendung von mikrobiziden Mitteln nach Anspruch 5 zur Bekämpfung von Mikroorganismen.

**Claims**

1. Trihaloallyl thiocyanates of the formula

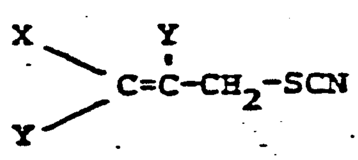

in which
X represents bromine, chlorine or iodine and
Y represents bromine or iodine.

2. Process for the preparation of trihaloallyl thiocyanates, characterised in that trihaloallyl compounds of the formula

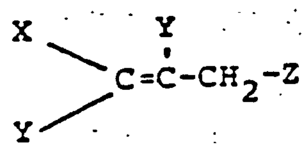

in which
X represents bromine, chlorine or iodine and
Y represents bromine or iodine and
Z represents a nucteofugic group,
are reacted with a thiocyanate of the formula
$M^{\ominus} SCN^{\ominus}$
in which
M denotes an alkali metal or ammonium.

3. Process according to Claim 2, characterised in that it is carried out in the temperature range from -20°C to 100°C.

4. Process according to Claims 2 and 3, characterised in that sodium iodide is used as a catalyst.

5. Microbicidal agents containing trihaloallyl thiocyanates of the formula

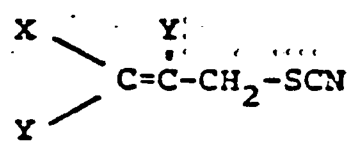

in which
X represents bromine, chlorine or iodine and
Y represents bromine or iodine.

6. Use of microbicidal agents according to Claim 5 for combating microorganisms.

**Revendications**

1. Thiocyanates de trihalogénoallyle de formule

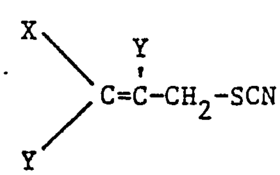

dans laquelle
X représente le brome, le chlore ou l'iode, et
Y représente le brome ou l'iode.

2. Procédé de préparation de thiocyanate de trihalogénoallyle, caractérisé en ce que l'on fait réagir des composés trihalogénoallyliques de formule

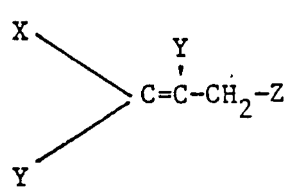

dans laquelle
X représente le chlore ou l'iode, et
Y représente le brome ou l'iode, et
Z représente un groupe nucléofuge,
avec un thiocyanate de formule
$M^{\oplus} SCN^{\ominus}$
dans laquelle
M représente un métal alcalin ou l'ammonium.

3. Procédé selon la revendication 2, caractérisé en ce qu'on le met en oeuvre dans l'intervalle de température de -20 à 100° C.

4. Procédé selon les revendications 2 et 3, caractérisé en ce que l'on utilise l'iodure de sodium en tant que catalyseur.

5. Produits microbicides contenant des thiocyanates de trihalogénoallyle de formule

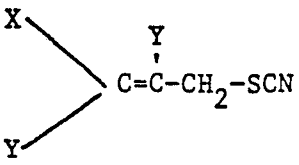

dans laquelle
X représente le brome, le chlore ou l'iode, et
Y représente le brome ou l'iode.

6. Utilisation des produits microbicides selon la revendication 5, dans la lutte contre les micro-organismes.